# EUROPÄISCHE PATENTSCHRIFT

(11) **EP 1 910 475 B1**
(45) Veröffentlichungstag und Bekanntmachung des Hinweises auf die Patenterteilung: **08.04.2009**
(21) Anmeldenummer: 06754267.0
(22) Anmeldetag: 09.06.2006
(51) Int. Cl.: C09C 1/00

(54) **MISCHUNG AUS INTERFERENZPIGMENTEN**
MIXTURE OF INTERFERENCE PIGMENTS
MELANGE DE PIGMENTS D'INTERFERENCE

(30) Priorität: 29.06.2005 DE 102005030244
(43) Veröffentlichungstag der Anmeldung: 16.04.2008
(73) Patentinhaber: Merck Patent GmbH, 64293 Darmstadt (DE)
(72) Erfinder: KNIESS, Helge, Bettina, 64380 Rossdorf (DE); PFAFF, Gerhard, 64839 Münster (DE)
(86) Internationale Anmeldenummer: PCT/EP2006/005566
(87) Internationale Veröffentlichungsnummer: WO 2007/000233

(56) Entgegenhaltungen:
- EP-A- 0 327 739
- EP-A- 0 450 945
- EP-A- 1 624 030
- DE-A1- 4 141 069
- DE-A1- 10 331 903

## Beschreibung

Die vorliegende Erfindung betrifft eine Mischung aus Interferenzpigmenten, wobei die Mischung mindestens ein Interferenzpigment A umfassend ein Substrat und eine metallisches Eisen enthaltende Beschichtung und ein Interferenzpigment B umfassend ein Substrat und eine Beschichtung enthaltend metallisches Zinn und zusätzlich mindestens ein Metalloxid enthält, deren Verwendung in Kosmetika, Lacken, Farben, Kunststoffen, Folien, im Sicherheitsdruck, in Sicherheitsmerkmalen in Dokumenten und Ausweisen, zur Saatguteinfärbung, zur Lebensmitteleinfärbung, zur Lasermarkierung oder in Arzneimittelüberzügen sowie zur Herstellung von Pigmentpräparationen und Trockenpräparaten sowie Kosmetika, Lacke, Farben, Kunststoffe, Folien, Dokumente und Ausweise, Saatgut, Lebensmittel oder Arzneimittelüberzüge, Pigmentpräparationen und Trockenpräparate enthaltend die erfindungsgemäße Mischung.

Mischungen von Interferenzpigmenten sind dem Fachmann bekannt und finden sich beispielsweise in unterschiedlichen Lackformulierungen im Bereich der Automobillackierungen.

Interferenzpigmente, und damit auch Mischungen dieser Pigmente, zeigen in Lackanwendungen oft nicht das gewünschte Deckvermögen, insbesondere dann, wenn sie auf semitransparenten oder transparenten Substraten, wie z.B. Glimmer, SiO₂ oder Al₂O₃ beruhen. Um ein ausreichendes Deckvermögen zu gewährleisten, wird den Pigmentmischungen üblicherweise Ruß zugesetzt. Die Zumischung von Ruß hat aber den Nachteil, dass der Ruß aufgrund seines sehr starken Deckvermögens nur schlecht dosierbar ist. Nur eine geringe Überdosierung führt zu einer sehr starken Erhöhung des Deckvermögens, die wiederum dazu führt, dass zur Erzielung eines gewünschten Farbeindrucks eine gewollte gestalterische Einbeziehung des Untergrunds, auf den der Lack appliziert wird, stark erschwert, wenn nicht gänzlich unmöglich gemacht wird. Die Einstellung eines vorgegebenen Deckvermögens für den aufzubringenden Lack ist damit nur schwer steuerbar.

Es bestand daher die Aufgabe Mischungen, insbesondere für Lackanwendungen, bereitzustellen, die die Einstellung eines vorgegebenen Deckvermögens erlauben und dabei attraktive optische Effekte zeigen.

Diese Aufgabe wird durch Mischungen gemäß der vorliegenden Erfindung erfüllt. Gegenstand der vorliegenden Erfindung ist demnach eine Mischung aus Interferenzpigmenten, wobei die Mischung mindestens ein Interferenzpigment A umfassend ein Substrat und eine metallisches Eisen enthaltende Beschichtung und ein Interferenzpigment B umfassend ein Substrat und eine Beschichtung enthaltend metallisches Zinn und zusätzlich mindestens ein Metalloxid enthält.

Mischungen gemäß der vorliegenden Erfindung zeichnen sich dadurch aus, dass das Deckvermögen, z.B. eines Lacks, gut gesteuert werden kann, das heißt ein gewünschter optischer Effekt kann unter Einbeziehung des Untergrunds, z.B. unter Einbeziehung des Farbeindrucks des Gegenstands oder vorherig aufgebrachter farbgebender Schichten, einfacher und genauer eingestellt werden oder sogar überhaupt erst realisiert werden. Darüber hinaus wurde überraschenderweise gefunden, dass die Mischung gemäß der vorliegenden Erfindung im Vergleich zu Mischungen enthaltend Ruß eine besonders gute Winkelabhängigkeit des Deckvermögens aufweist. So wird bei Betrachtung aus flachen Winkeln von mit der erfindungsgemäßen Mischung beschichteter Oberflächen eine höhere Transparenz beobachtet als bei Betrachtung aus steileren Winkeln. Dies führt insbesondere bei Dreischichtlackierungen, wie sie beispielsweise im Automobilbereich verwendet werden, zu besonders attraktiven Effekten. Mischungen gemäß der vorliegenden Erfindung zeichnen sich desweiteren dadurch aus, dass sie zur Ergänzung der gewünschten Effekte im Anwendungssystem ganz ohne Ruß bzw. mit, im Vergleich zu üblichen Anwendungen, sehr geringen Rußzusätzen auskommen. Da der Zusatz von Ruß stets mit einem Glanzverlust des Interferenzpigmentes einhergeht, können mit den erfindungsgemäßen Mischungen hochglänzende Farbeffekte, die gleichzeitig ein hohes Deckvermögen aufweisen, erreicht werden.

Wegen der oben genannten Vorteile sind die erfindungsgemäßen Pigmentmischungen vielseitig einsetzbar. Gegenstand der vorliegenden Erfindung sind demgemäss auch die Verwendung der Pigmentmischungen in Kosmetika, Lacken, Farben, Kunststoffen, Folien, im Sicherheitsdruck, in Sicherheitsmerkmalen in Dokumenten und Ausweisen, zur Saatguteinfärbung, zur Lebensmitteleinfärbung, zur Lasermarkierung oder in Arzneimittelüberzügen sowie zur Herstellung von Pigmentpräparationen und Trockenpräparaten sowie Kosmetika, Lacke, Farben, Kunststoffe, Folien, Dokumente und Ausweise, Saatgut, Lebensmittel oder Arzneimittelüberzüge Pigmentpräparationen und Trockenpräparate enthaltend Pigmentmischungen gemäß der vorliegenden Erfindung.

In der erfindungsgemäßen Pigmentmischung werden Interferenzpigmente umfassend ein Substrat und eine metallisches Eisen enthaltende Beschichtung und ein Interferenzpigmente umfassend ein Substrat und eine Beschichtung enthaltend metallisches Zinn und zusätzlich mindestens ein Metalloxid miteinander kombiniert, um die oben genannten Effekte zu erzielen. Das Verhältnis von Interferenzpigment A zu Interferenzpigment B wann an die jeweiligen Erfordernisse angepasst werden und beträgt üblicherweise 99:1 bis 1:99 Bevorzugt ist das Verhältnis von Interferenzpigment A zu Interferenzpigment B 10:1 bis 0.5:1 Diese bevorzugten Mischungen sind besonders vorteilhaft, weil sie je nach zu erzielenden Anwendungseigenschaften ein Optimum an Glanz, Deckvermögen und Winkelabhängigkeit des Deckvermögens darstellen.

Prinzipiell können die Interferenzpigmente A und B jede denkbare Form aufweisen, vorzugsweise sind sie aber plättchenförmig. Die Größe der Interferenzpigmente ist an sich nicht kritisch. Plättchenförmige Interferenzpigmente A und B weisen in der Regel eine Dicke zwischen 0.05 und 5 µm, insbesondere zwischen 0.1 und 4.5 µm auf. Die Ausdehnung der Interferenzpigmente in der Länge bzw. Breite kann zwischen 1 und 250 µm betragen, vorzugsweise liegt sie im Bereich von 2 bis 200 µm und ganz besonders bevorzugt im Bereich von 2 bis 100 µm. Die Größe der Substrate kann an die Anforderungen der jeweiligen Applikationen angepasst werden.

Bei den Interferenzpigmenten A handelt es sich Interferenzpigmente umfassend ein Substrat und eine metallisches Eisen enthaltende Beschichtung. Die in der Mischung eingesetzten Interferenzpigmente B umfassen ein Substrat und eine Beschichtung enthaltend metallisches Zinn und zusätzlich mindestens ein Metalloxid.

Das Substrat der Interferenzpigmente A und B ist voneinander unabhängig und kann synthetischen oder natürlichen Glimmer, Schichtsilikate, Glas, Borosilikate, SiO₂, Al₂O₃, TiO₂, Graphit, und/oder BiOCl umfassen. Das bedeutet, dass die Interferenzpigmente A und B auf unterschiedlichen Substraten basieren können.

Bei den in der erfindungsgemäßen Mischung eingesetzten Interferenzpigmenten A und B können zwischen der metallisches Eisen enthaltenden Beschichtung und/oder zwischen der metallisches Zinn und zusätzlich mindestens ein Metalloxid enthaltenden Beschichtung und dem Substrat in einer weiteren Ausführungsform der vorliegenden Erfindung eine oder mehrere Schichten enthaltend Metalloxide, Metalloxidhydrate, Metallsuboxide, Metalle, Metallfluoride, Metallnitride, Metalloxynitride und/oder Mischungen hieraus vorliegen.

Die Metalloxid-, Metalloxidhydrat, Metallsuboxid-, Metall-, Metallfluorid-, Metallnitrid-, Metalloxynitridschichten oder die Mischungen hieraus können niedrig- (Brechzahl < 1.8) oder hochbrechend (Brechzahl ≥ 1.8) sein. Als Metalloxide und Metalloxidhydrate eignen sich alle als Schichten aufzubringende Metalloxide oder Metalloxidhydrate, wie z.B. Aluminiumoxid, Aluminiumoxidhydrat, Eisenoxid, Zinnoxid, Ceroxid, Zinkoxid, Zirkoniumoxid, Chromoxid, Titanoxid, insbesondere Titandioxid, Titanoxidhydrat sowie Mischungen hieraus, wie z.B. Ilmenit oder Pseudobrookit. Als Metallsuboxide können beispielsweise die Titansuboxide eingesetzt werden. Als Metalle eignen sich z.B. Eisen, Chrom, Aluminium, Nickel, Silber, Gold, Titan, Kupfer oder Legierungen, als Metallfluorid eignet sich beispielsweise Magnesiumfluorid. Als Metallnitride oder Metalloxynitride können beispielsweise die Nitride oder Oxynitride der Metalle Titan, Zirkonium und/oder Tantal eingesetzt werden. Bevorzugt sind Metalloxid-, Metall-, Metallfluorid und/oder Metalloxidhydratschichten und ganz besonders bevorzugt Metalloxid- und/oder Metalloxidhydratschichten auf den Substraten aufgebracht. Weiterhin können auch Mehrschichtaufbauten aus hoch- und niedrigbrechenden Metalloxid-, Metalloxidhydrat-, Metall- oder Metallfluoridschichten vorliegen, wobei sich vorzugsweise hoch- und niedrigbrechende Schichten abwechseln.

Besonders geeignete Materialien mit hoher Brechzahl sind beispielsweise TiO₂, ZrO₂, ZnO, SnO₂ und/oder Mischungen hieraus. Besonders bevorzugt ist TiO₂. Die Dicke dieser Schichten beträgt dabei jeweils etwa 3 bis 300 nm und bevorzugt 20 bis 200 nm.

Besonders geeignete Materialien mit niedriger Brechzahl sind beispielsweise SiO₂, SiO(OH)₂, Al₂O₃, AIO(OH), B₂O₃, MgF₂ und/oder Mischungen hieraus. Besonders bevorzugt ist SiO₂. Die Dicke der einzelnen Schichten aus diesen Materialien beträgt zwischen 3 und 300 nm, bevorzugt sind sie dicker als 20 nm und bis zu 200 nm dick.

Insgesamt sind die Materialien der zusätzlichen Schichten so auszuwählen und deren Schichtdicken in Abhängigkeit des Schichtmaterials in der Weise einzustellen, dass die Semitransparenz der Interferenzpigmente A und B erhalten bleibt.

Bei den Interferenzpigmenten A sind die genannten Substrate mit einer Eisen-haltigen Beschichtung umfassend metallisches Eisen versehen, die als äußere optisch aktive Schicht wirkt. Die Schichtdicke der Eisen-haltigen Beschichtung beträgt 1 bis 300 nm, vorzugsweise 1 bis 100 nm. Der Anteil an metallischen Eisen in der Eisen-haltigen Beschichtung beträgt 10 bis 100 Gew.-%, vorzugsweise 30 bis 99 Gew.-% und insbesondere 60 bis 85 Gew.-%, bezogen auf die Eisen-haltige Beschichtung. Ganz besonders bevorzugt liegt das metallische Eisen in der Eisen-haltigen Beschichtung in Kombination mit FeO und/oder Fe₃O₄ vor. Darüber hinaus können in der Eisen-haltigen Beschichtung auch weitere einfache oder komplexe Metalloxide vorliegen, beispielsweise TiO₂, Ilmenit oder Pseudobrookit. Pigmente dieses Typs sind erhältlich durch Umsetzung eines mit einer Eisenoxid-haltigen Beschichtung beschichteten Substrates in einem reduzierenden Gasgemisch aus Stickstoff und Wasserstoff unter Bildung von metallischem Eisen. Dies ermöglicht die Bereitstellung der Eisen-haltigen Beschichtung enthaltend metallisches Eisen.

Bei den Interferenzpigmenten B sind die genannten Substrate mit einer Beschichtung enthaltend metallisches Zinn und zusätzlich mindestens ein Metalloxid versehen, die als äußere optisch aktive Schicht wirkt. Die Schichtdicke der Beschichtung enthaltend metallisches Zinn und zusätzlich mindestens ein Metalloxid beträgt 1 bis 300 nm, vorzugsweise 1 bis 100 nm. Der Anteil an metallischem Zinn in der Beschichtung beträgt 0.01 bis 50 Gew.-%, vorzugsweise 0.05 bis 20 Gew.-% und insbesondere 0.1 bis 10 Gew.-%, bezogen auf die Beschichtung. Als zusätzlich mindestens ein Metalloxid eignen sich Zinnoxid, Titanoxid, Zirkoniumoxid und Zinkoxid.

Ganz besonders bevorzugt liegt das metallische Zinn in der Beschichtung in Kombination mit Titanoxid als zusätzliches Metalloxid vor. Darüber hinaus können in der Zinn-haltigen Beschichtung auch weitere einfache oder komplexe Metalloxide vorliegen, beispielsweise Ilmenit oder Pseudobrookit.

Pigmente dieses Typs sind erhältlich durch Umsetzung eines mit einer Zinndioxid-haltigen und optional zusätzlich mindestens ein weiteres Metalloxid enthaltenden Beschichtung beschichteten Substrates in einem reduzierenden Gasgemisch aus Stickstoff und Wasserstoff unter Bildung von metallischem Zinn. Dies ermöglicht die Bereitstellung der Beschichtung enthaltend metallisches Zinn und zusätzlich mindestens ein Metalloxid.

Das für die Herstellung der Interferenzpigmente A und B einzusetzende reduzierende Gasgemisch aus Stickstoff und Wasserstoff hat einen Anteil von Wasserstoff im Bereich von 2.5 bis 25 Vol.-%, insbesondere von 4 bis 10 Vol.-%, und ganz besonders bevorzugt von 5 bis 8 Vol.-%.

Die Reduktion der Eisenoxid-haltigen Beschichtung beziehungsweise der Zinndioxid-haltigen und optional zusätzlich mindestens ein weiteres Metalloxid enthaltenden Beschichtung erfolgt bei Temperaturen von 400 bis 1200°C, vorzugsweise von 500 bis 1000°C und insbesondere bevorzugt von 550 bis 900°C. Die Glühdauer beträgt 15-240 Minuten, vorzugsweise 30-120 Minuten und insbesondere 30-90 Minuten

In einer weiteren Ausführungsform der vorliegenden Erfindung können die Interferenzpigmente A und B weiterhin mit einer zusätzlichen organischen Beschichtung als äußere Schicht versehen sein. Beispiele für derartige Beschichtungen finden sich z.B. in EP 0 632 109, US 5,759,255, DE 43 17 019, DE 39 29 423, DE 32 35 017, EP 0 492 223, EP 0 342 533, EP 0 268 918, EP 0 141 174, EP 0 764 191, WO 98/13426 oder EP 0 465. 805, Interferenzpigmente enthaltend diese organische Beschichtung, z.B. aus Organosilanen oder Organotitanaten bzw. Organozirkonaten zeigen neben den bereits genannten verbesserten optischen Eigenschaften zusätzlich eine erhöhte Stabilität gegenüber Witterungseinflüssen, wie z.B. Feuchtigkeit und Licht, was vor allem für Industrielacke und im Automobilbereich von besonderem Interesse ist. Der Verfahrensschritt der Aufbringung der organischen Beschichtung kann direkt an die Schritte zur Herstellung der Pigmente angeschlossen werden, oder unabhängig davon durchgeführt werden. Die hierbei aufgebrachten Stoffe umfassen lediglich einen Gewichtsanteil von 0.1 bis 5 Gew. %, vorzugsweise 0.5 bis 3 Gew. %, des gesamten Pigmentes.

Die Herstellung der erfindungsgemäßen Pigmentmischung erfolgt durch Mischen der Interferenzpigmente A und B, wobei das Mischen auf allen dem Fachmann bekannten Arten erfolgen kann, z.B. durch einfaches Einrühren in das Anwendungssystem. Ein aufwendiges Mahlen und Dispergieren der Pigmente ist nicht erforderlich.

Die erfindungsgemäßen Pigmentmischungen eignen sich aufgrund ihrer vorteilhaften Eigenschaften für eine große Bandbreite an Anwendungen. Gegenstand der Erfindung ist daher auch die Verwendung der erfindungsgemäßen Pigmentmischungen in Kosmetika, Lacken, Farben, Kunststoffen, Folien, im Sicherheitsdruck, in Sicherheitsmerkmalen in Dokumenten und Ausweisen, zur Saatguteinfärbung, zur Lebensmitteleinfärbung, zur Lasermarkierung oder in Arzneimittelüberzügen sowie zur Herstellung von Pigmentpräparationen und Trockenpräparaten.

Im Falle von Kosmetika eignen sich die erfindungsgemäßen Pigmentmischungen besonders für Produkte der dekorativen Kosmetik, wie z.B. Nagellacke, farbgebende Puder, Lippenstifte oder Lidschatten. Selbstverständlich können die erfindungsgemäßen Pigmentmischungen in den Formulierungen auch mit jeder Art von kosmetischen Roh- und Hilfsstoffen kombiniert werden. Dazu gehören u.a. Öle, Fette, Wachse, Filmbildner, Konservierungsmittel und allgemein anwendungstechnische Eigenschaften bestimmende Hilfsstoffe, wie z.B. Verdicker und rheologische Zusatzstoffe wie etwa Bentonite, Hektorite, Siliziumdioxid, Ca-Silikate, Gelatine, hochmolekulare Kohlenhydrate und/oder oberflächenaktive Hilfsmittel, etc. Die erfindungsgemäßen Pigmentmischungen enthaltenden Formulierungen können dem lipophilen, hydrophilen oder hydrophoben Typ angehören. Bei heterogenen Formulierungen mit diskreten wässrigen und nicht-wässrigen Phasen können die erfindungsgemäßen Partikel in jeweils nur einer der beiden Phasen enthalten oder auch über beide Phasen verteilt sein.

Die pH-Werte der wässrigen Formulierungen können zwischen 1 und 14, bevorzugt zwischen 2 und 11 und besonders bevorzugt zwischen 5 und 8 liegen. Den Konzentrationen der erfindungsgemäßen Pigmentmischungen in der Formulierung sind keine Grenzen gesetzt. Sie können - je nach Anwendungsfall - zwischen 0,001 (rinse-off-Produkte, z.B. Duschgele) - 99% (z.B. Glanzeffekt-Artikel für besondere Anwendungen) liegen. Die erfindungsgemäßen Pigmentmischungen können weiterhin auch mit kosmetischen Wirkstoffen kombiniert werden. Geeignete Wirkstoffe sind z.B. Insect Repellents, UV A/BC-Schutzfilter (z.B. OMC, B3, MBC), Anti-Ageing-Wirkstoffe, Vitamine und deren Derivate (z.B. Vitamin A, C, E etc.), Selbstbräuner (z.B. DHA, Erythrolose u.a.) sowie weitere kosmetische Wirkstoffe wie z.B. Bisabolol, LPO, Ectoin, Emblica, Allantoin, Bioflavanoide und deren Derivate.

Bei Einsatz der Pigmentmischungen in Lacken und Farben sind alle dem Fachmann bekannten Anwendungsbereiche möglich, wie z.B. Pulverlacke, Automobillacke, Druckfarben für den Tief-, Offset-, Sieb- oder Flexodruck sowie für Lacke in Außenanwendungen. Die Lacke und Farben können hierbei beispielsweise strahlungshärtend, physikalisch trocknend oder chemisch härtend sein. Für die Herstellung der Druckfarben oder Flüssiglacke ist eine Vielzahl von Bindern, z.B. auf der Basis von Acrylaten, Methacrylaten, Polyestern, Polyurethanen, Nitrocellulose, Ethylcellulose, Polyamid, Polyvinylbutyrat, Phenolharzen, Maleinharzen, Stärke oder Polyvinylalkohol, Aminharzen, Alkydharzen, Epoxidharzen, Polytetrafluorethylen, Polyvinylidenfluoriden, Polyvinylchlorid oder Mischungen hieraus geeignet, insbesondere wasserlösliche Typen. Bei den Lacken kann es sich um Pulverlacke oder wasser- oder lösemittelbasierte Lacke handeln, wobei die Auswahl der Lackbestandteile dem Allgemeinwissen des Fachmanns unterliegt. Gängige polymere Bindemittel für Pulverlacke sind beispielsweise Polyester, Epoxide, Polyurethane, Acrylate oder Mischungen hieraus.
Grundsätzlich können die erfindungsgemäßen Pigmentmischungen in den Formulierungen auch mit jeder Art von anderen Farbmitteln oder Füllstoffen kombiniert eingesetzt werden.

Darüber hinaus können die erfindungsgemäßen Pigmentmischungen zur Pigmentierung von Folien und Kunststoffen verwendet werden, so z.B. für Agrarfolien, Geschenkfolien, Kunststoffbehältnisse und Formkörper für alle dem Fachmann bekannten Anwendungen. Als Kunststoffe eignen sich alle gängigen Kunststoffe für die Einarbeitung der erfindungsgemäßen Pigmentmischungen, z.B. Duromere oder thermoplastische Kunststoffe. Die Beschreibung der Anwendungsmöglichkeiten und der einsetzbaren Kunststoffe, Verarbeitungsverfahren und Additive finden sich z.B. in der RD 472005 oder in R. Glausch, M. Kieser, R. Maisch, G. Pfaff, J. Weitzel, Perlglanzpigmente, Curt R. Vincentz Verlag, 1996, 83 ff., deren Offenbarungsgehalt hier mit umfasst ist.

Darüber hinaus eignen sich die erfindungsgemäßen Pigmentmischungen auch für den Einsatz im Sicherheitsdruck und in sicherheitsrelevanten Merkmalen für z.B. fälschungssichere Karten und Ausweise, wie z.B. Eintrittskarten, Personalausweise, Geldscheine, Schecks und Scheckkarten sowie für andere fälschungssichere Dokumente. Im Bereich der Landwirtschaft können die Pigmentmischungen zur Einfärbung von Saatgut und anderen Ausgangsgütern verwendet werden, darüber hinaus im Lebensmittelbereich zur Pigmentierung von Lebensmitteln. Zur Pigmentierung von Überzügen in Arzneimitteln wie z.B. Tabletten oder Dragees sind die erfindungsgemäßen Pigmentmischungen ebenfalls einsetzbar.

Für die Lasermarkierung unter Verwendung der erfindungsgemäßen Mischung können alle bekannten thermoplastischen Kunststoffe, wie sie z.B. im Ullmann, Bd. 15, S. 457 ff., Verlag VCH beschrieben werden, verwendet werden. Geeignete Kunststoffe sind z.B. Polyethylen, Polypropylen, Polyamide, Polyester, Polyesterester, Polyetherester, Polyphenylenether, Polyacetal, Polybutylenterephthalat, Polymethylacrylat, Polyvinylacetat, Polystyrol, Acrylnitril-Butadien-Styrol, Acrylnitril-Styrol-Acrylester, Polycarbonat, Polyethersulfone, Polyetherketone sowie deren Copolymere und/oder Mischungen.

Die Einarbeitung der erfindungsgemäßen Mischung in den thermoplastischen Kunststoff erfolgt, indem das Kunststoffgranulat mit der Mischung gemischt und dann unter Wärmeeinwirkung verformt wird. Dem Kunststoffgranulat können bei der Einarbeitung der Mischung dem Fachmann bekannte Haftmittel, organische polymerverträgliche Lösemittel, Stabilisatoren und/oder unter den Arbeitsbedingungen temperaturstabile Tenside zugesetzt werden. Die Herstellung der pigmentierten Kunststoffgranulate erfolgt in der Regel so, dass in einem geeigneten Mischer das Kunststoffgranulat vorgelegt, mit eventuellen Zusätzen benetzt und danach die erfindungsgemäße Mischung zugesetzt und untergemischt wird. Die so erhaltene Mischung kann dann direkt in einem Extruder oder einer Spritzgussmaschine verarbeitet werden. Anschließend findet die Markierung mit geeigneter Strahlung statt.

Vorzugsweise wird bei der Markierung energiereiche Strahlung eingesetzt, im allgemeinen im Wellenlängenbereich von 157 bis 10600 nm, insbesondere im Bereich von 300 bis 10600 nm. Beispielsweise seien hier CO₂-Laser (10600 nm), Nd:YAG-Laser (1064 bzw. 532 nm) oder gepulste UV-Laser (Excimer-Laser) erwähnt. Die Excimerlaser weisen folgende Wellenlängen auf: F2-Excimerlaser (157 nm), ArF-Excimerlaser (193 nm), KrCl-Excimerlaser (222 nm), KrF-Excimerlaser (248 nm), XeCl-Excimerlaser (308 nm), XeF-Excimerlaser (351 nm), frequenzvervielfachte Nd : YAG-Laser mit Wellenlängen von 355 nm (frequenzverdreifacht) oder 265 nm (frequenzvervierfacht). Besonders bevorzugt werden Nd : YAG-Laser (1064 bzw. 532 nm) und CO₂-Laser eingesetzt. Die Energiedichten der eingesetzten Laser liegen im allgemeinen im Bereich von 0,3 mJ/cm² bis 50 J/cm², vorzugsweise 0,3 mJ/cm² bis 10 J/cm².

Die Beschriftung mit dem Laser erfolgt derart, dass der Probenkörper in den Strahlengang eines gepulsten Lasers, vorzugsweise eines CO₂- oder Nd : YAG-Lasers gebracht wird. Ferner ist eine Beschriftung mit einem Excimer-Laser, z. B. über eine Maskentechnik, möglich. Jedoch sind auch mit anderen herkömmlichen Lasertypen, die eine Wellenlänge in einem Bereich hoher Absorption der verwendeten laserlichtabsorbierenden Substanz aufweisen, die gewünschten Ergebnisse zu erzielen. Die erhaltene Markierung wird durch die Bestrahlungszeit (bzw. Pulszahl bei Pulslasern) und Bestrahlungsleistung des Lasers sowie des verwendeten Kunststoffsystems bzw. Lacksystems bestimmt. Die Leistung der verwendeten Laser hängt von der jeweiligen Anwendung ab und kann im Einzelfall vom Fachmann ohne weiteres ermittelt werden.

Bei der Verwendung von gepulsten Lasern liegt die Pulsfrequenz im allgemeinen im Bereich von 1 bis 30 kHz. Entsprechende Laser, die im erfindungsgemäßen Verfahren eingesetzt werden können, sind kommerziell erhältlich.

Die Verwendung der erfindungsgemäßen Mischung zur Lasermarkierung kann in allen oben genannten Kunststoffen erfolgen. Die auf diese Weise pigmentierten Kunststoffe können als Formkörper in der Elektro-, Elektronik- und Kraftfahrzeugindustrie Anwendung finden. Ein weiteres wichtiges Anwendungsgebiet für die Laserbeschriftung sind Ausweiskarten und Kunststoffmarken zur individuellen Kennzeichnung von Tieren. Der Anteil an Interferenzpigmenten im Kunststoff beträgt im Falle der Lasermarkierung bei den Anwendungen 0.01 bis 10 Gew.-%, vorzugsweise 0.05 bis 5 Gew.-% und insbesondere 0.1 bis 3 Gew.-%. Die Kennzeichnung und Beschriftung von Gehäusen, Leitungen, Tastenkappen, Zierleisten bzw. Funktionsteilen im Heizungs-, Lüftungs- und Kühlbereich oder Schalter, Stecker, Hebel und Griffe, die aus den mit den erfindungsgemäßen Pigmenten pigmentierten Kunststoffen bestehen, kann selbst an schwer zugänglichen Stellen mit Hilfe von Laserlicht erfolgen. Die Markierungen zeichnen sich dadurch aus, dass sie wisch- und kratzfest, stabil bei nachträglichen Sterilisationsprozessen und hygienisch rein beim Markierungsprozeß aufbringbar sind.

Die erfindungsgemäßen Pigmentmischungen eignen sich in den oben genannten Anwendungsgebieten ebenso zur Verwendung in Abmischungen mit allen bekannten organischen oder anorganischen Farbstoffen und/oder Pigmenten. Organische Pigmente und Farbstoffe sind beispielsweise Monoazopigmente, Disazopigmente, polycyclische Pigmente, kationische, anionische oder nichtionische Farbstoffe. Anorganische Farbstoffe und Pigmente sind beispielsweise Weisspigmente, Buntpigmente, Schwarzpigmente oder Effektpigmente. Beispiele für geeignete Effektpigmente sind Metalleffektpigmente, Perlglanzpigmente oder Interferenzpigmente, die in der Regel auf ein- oder mehrfach beschichteten Plättchen auf Basis von Glimmer, Glas, Al₂O₃, Fe₂O₃, SiO₂, etc. beruhen. Beispiele für Aufbauten und besondere Eigenschaften der genannten Pigmente finden sich beispielsweise in RD 471001 oder RD 472005, deren Offenbarung hiermit unter Bezugnahme in der vorliegenden Erfindung mit eingeschlossen ist. Darüber hinaus eignen sich als weitere Farbmittel zur Abmischung mit den erfindungsgemäßen Mischungen lumineszierende Farbstoffe und/oder Pigmente sowie holographische Pigmente oder LCPs (Liquid Crystal Polymers). Die erfindungsgemäßen Pigmentmischungen können in jedem Verhältnis mit handelsüblichen Pigmenten und Füllern gemischt werden.

Als Füllstoffe sind z.B. natürlicher und synthetischer Glimmer, Nylon Powder, reine oder gefüllte Melaminharze, Talcum, Gläser, Kaolin, Oxide oder Hydroxide von Aluminium, Magnesium, Calcium, Zink, BiOCl, Bariumsulfat, Calciumsulfat, Calciumcarbonat, Magnesiumcarbonat, Kohlenstoff, sowie physikalische oder chemische Kombinationen dieser Stoffe zu nennen. Bezüglich der Partikelform des Füllstoffes gibt es keine Einschränkungen; sie kann den Anforderungen gemäß z.B. plättchenförmig, sphärisch oder nadelförmig sein.

Die erfindungsgemäßen Pigmentmischungen sind weiterhin geeignet zur Herstellung von fließfähigen Pigmentpräparationen und Trockenpräparaten enthaltend ein oder mehrere erfindungsgemäße Partikel, Bindemittel und optional ein oder mehrere Additive. Unter Trockenpräparate sind auch Präparate zu verstehen, die 0 bis 8 Gew.-%, vorzugsweise 2 bis 8 Gew.-%, insbesondere 3 bis 6 Gew.-%, an Wasser und/oder eines Lösemittels oder Lösemittelgemisches enthalten. Die Trockenpräparate liegen vorzugsweise als Pellets, Granulate, Chips, Würstchen oder Briketts vor und weisen Teilchengrößen von 0.2-80 mm auf. Die Trockenpräparate finden insbesondere Anwendung bei der Herstellung von Druckfarben und in kosmetischen Formulierungen.

Kosmetika, Lacke, Farben, Kunststoffe, Folien, Dokumente und Ausweise, Saatgut, Lebensmittel oder Arzneimittelüberzüge, Pigmentpräparationen und Trockenpräparate enthaltend die erfindungsgemäßen Pigmentmischungen sind ebenfalls Gegenstand der vorliegenden Erfindung.

Die nachfolgenden Beispiele sollen die Erfindung näher erläutern, ohne sie jedoch zu begrenzen.

### Beispiele:

### a) Herstellung von Interferenzpigment A

100 g Glimmer der Teilchengröße 10-60 µm werden in 1.9 entmineralisiertem Wasser unter Rühren auf 75°C erhitzt.
Der pH-Wert der Suspension wird mit 10 %-iger Salzsäure auf 3.0 eingestellt. Nun werden 200 g einer 30 %-igen FeCl₃-Lösung zudosiert, wobei der pH-Wert durch gleichzeitiges Zutropfen einer 32 %-igen Natronlauge konstant gehalten wird. Das Produkt wird abfiltriert, gewaschen, getrocknet und bei 700°C in einem Gasgemisch aus Stickstoff und Wasserstoff (Anteil an Wasserstoff: 8 Vol.-%) reduziert. Man erhält ein glänzendes Pigment mit silberner Interferenz, grauschwarzer Körperfarbe und hohem Glanz, dessen Beschichtung aus 74 Gew.-% metallischen Eisens besteht.

### b) Herstellung von Interferenzpigment B

100 g Glimmer der Teilchengröße 10-60 µm werden in 1.91 entmineralisiertem Wasser unter Rühren auf 75°C erhitzt.
Mit einer 5 %-igen Salzsäure wird der pH-Wert der Suspension auf 1.8 eingestellt. Es folgt das Zudosieren einer Zinntetrachloridlösung (aus 3 g SnCl₄ · 5 H₂O und 10 ml konzentrierter Salzsäure in 90 ml entmineralisiertem Wasser), wobei der pH-Wert durch gleichzeitiges Zutropfen einer 32 %-igen Natronlauge konstant gehalten wird. Danach wird eine 30 %-ige Titantetrachloridlösung (180 g TiCl₄-Lösung w = 60%, gelöst in 180 g entmineralisiertem Wasser), wobei der pH-Wert durch gleichzeitiges Zutropfen einer 32 %-igen Natronlauge konstant gehalten wird. Das Produkt wird abfiltriert, gewaschen, getrocknet und bei 850°C in einem Gasgemisch aus Stickstoff und Wasserstoff (Anteil an Wasserstoff: 8 Vol.-%) reduziert.Man erhält ein metallisches Zinn enthaltendes Pigment mit silberner Interferenz, farbneutraler grauer Körperfarbe und hohem Glanz.

### c) Herstellung einer Pigmentmischung

Die Pigmente aus Beispiel a) und b) werden im massebezogenen Verhältnis von 1:0.75 (Interferenzpigment A:Interferenzpigment B) gemischt. Die so erhaltene Mischung wird in einen Acrylat-Melamin-Lack in einer Konzentration von 3.5 Gew.-% eingearbeitet und auf rote Lackbleche appliziert. Man erhält eine glänzende silberfarbene Beschichtung, welche im Abkippen einen leicht rötlichen Farbeindruck zeigt.

### Verpleichsbeispiel:

Das Pigment aus Beispiel b) wird mit Ruß im massebezogenen Verhältnis von 1:0.06 (Interferenzpigment B:Ruß) gemischt. Die so erhaltene Mischung wird in einen Acrylat-Melamin-Lack in einer Konzentration von 2.5 Gew.-% eingearbeitet und auf rote Lackbleche appliziert. Man erhält eine glänzende silberfarbene Beschichtung, bei der auch bei Betrachtung im flachen Winkel der rote Untergrund nicht mehr zu erkennen ist.

### Anwendungsbeispiel Lasermarkierung:

Ein PP-Granulat (PP-HD, Stamylan PPH 10 der Fa. DSM) wird durch Zusatz von 0.1 Gew.-% des Mischung aus Beispiel c) im Spritzguss verarbeitet. Das erhaltene Formteil (Plättchen) wird anschließend mit einem SHT-Nd:YAG-Laser beschriftet. Bei einer Pulsfrequenz von 2.5 kHz und einer Schreibgeschwindigkeit von 300 mm/s zeigen die Plättchen eine schwarze, kontrastreiche und abriebfeste Beschriftung. Mit steigender Energiedichte wird die Beschriftung zunehmend dunkler.

## Patentansprüche

1. Mischung aus Interferenzpigmenten, **dadurch gekennzeichnet, dass** die Mischung mindestens ein Interferenzpigment A umfassend ein Substrat und eine metallisches Eisen enthaltende Beschichtung und ein Interferenzpigment B umfassend ein Substrat und eine Beschichtung enthaltend metallisches Zinn und zusätzlich mindestens ein Metalloxid enthält.

2. Mischung gemäß Anspruch 1, **dadurch gekennzeichnet, dass** der Anteil an metallischem Eisen in der Eisen-haltigen Beschichtung 10 bis 100 Gew.-%, bezogen auf die Eisen-haltige Beschichtung, beträgt

3. Mischung gemäß Anspruch 1 oder 2, **dadurch gekennzeichnet, dass** das metallische Eisen in der Eisen-haltigen Beschichtung in Kombination mit FeO und/oder Fe₃O₄ vorliegt.

4. Mischung gemäß Anspruch 1, **dadurch gekennzeichnet, dass** der Anteil an metallischem Zinn in der Beschichtung enthaltend metallisches Zinn 0.01 bis 50 Gew.-%, bezogen auf die Beschichtung enthaltend metallisches Zinn und zusätzlich mindestens ein Metalloxid, beträgt.

5. Mischung gemäß Anspruch 1 oder 4, **dadurch gekennzeichnet, dass** das zusätzliche Metalloxid Titanoxid ist.

6. Mischung gemäß einem der Ansprüche 1 bis 5, **dadurch gekennzeichnet, dass** das Substrat von Interferenzpigment A und B unabhängig voneinander ist und synthetischen oder natürlichen Glimmer, Schichtsilikate, Glas, SiO₂, Al₂O₃, TiO₂, Graphit, und/oder BiOCl umfasst.

7. Mischung gemäß einem der Ansprüche 1 bis 6, **dadurch gekennzeichnet, dass** zwischen der metallisches Eisen enthaltenden Beschichtung und dem Substrat in Interferenzpigment A und/oder zwischen der metallisches Zinn und zusätzlich mindestens ein Metalloxid enthaltenden Beschichtung und dem Substrat in Interferenzpigment B zusätzlich eine oder mehrere Schichten enthaltend Metalloxide, Metalloxidhydrate, Metallsuboxide, Metalle, Metallfluoride, Metallnitride, Metalloxynitride und/oder Mischungen hieraus vorliegen.

8. Pigmentmischung gemäß einem der Ansprüche 1 bis 8, **dadurch gekennzeichnet, dass** das Verhältnis von Interferenzpigment A zu Interferenzpigment B 99:1 bis 1:99 beträgt.

9. Pigmentmischung gemäß einem der Ansprüche 1 bis 8, **dadurch gekennzeichnet, dass** die Interferenzpigmente A und B weiterhin mit einer zusätzlichen organischen Beschichtung als äußere Schicht versehen sind.

10. Verwendung von Mischungen gemäß Anspruch 1 in Kosmetika, Lacken, Farben, Kunststoffen, Folien, im Sicherheitsdruck, in Sicherheitsmerkmalen in Dokumenten und Ausweisen, zur Saatguteinfärbung, zur Lebensmitteleinfärbung, zur Lasermarkierung oder in Arzneimittelüberzügen sowie zur Herstellung von Pigmentpräparationen und Trockenpräparaten.

11. Verwendung gemäß Anspruch 10, **dadurch gekennzeichnet, dass** die Mischungen gemäß Anspruch 1 in Abmischung mit organischen oder anorganischen Farbstoffen und/oder Pigmenten vorliegen.

12. Kosmetika, Lacke, Farben, Kunststoffe, Folien, Dokumente und Ausweise, Saatgut, Lebensmittel oder Arzneimittelüberzüge, Pigmentpräparationen und Trockenpräparate enthaltend Mischungen gemäß Anspruch 1.

## Claims

1. Mixture of interference pigments, **characterised in that** the mixture comprises at least one interference pigment A comprising a substrate and a coating comprising metallic iron, and an interference pigment B comprising a substrate and a coating comprising metallic tin and in addition at least one metal oxide.

2. Mixture according to Claim 1, **characterised in that** the proportion of metallic iron in the iron-containing coating is 10 to 100% by weight, based on the iron-containing coating.

3. Mixture according to Claim 1 or 2, **characterised in that** the metallic iron is present in the iron-containing coating in combination with FeO and/or Fe₃O₄.

4. Mixture according to Claim 1, **characterised in that** the proportion of metallic tin in the coating comprising metallic tin is 0.01 to 50% by weight, based on the coating comprising metallic tin and in addition at least one metal oxide.

5. Mixture according to Claim 1 or 4, **characterised in that** the additional metal oxide is titanium oxide.

6. Mixture according to one of Claims 1 to 5, **characterised in that** the substrates of interference pigments A and B are independent of one another and comprise synthetic or natural mica, phyllosilicates, glass, SiO₂, Al₂O, TiO₂, graphite and/or BiOCl.

7. Mixture according to one of Claims 1 to 6, **characterised in that** one or more layers comprising metal oxides, metal oxide hydrates, metal suboxides, metals, metal fluorides, metal nitrides, metal oxynitrides and/or mixtures thereof are additionally present between the coating comprising metallic iron and the substrate in interference pigment A and/or between the coating comprising metallic tin and in addition at least one metal oxide and the substrate in interference pigment B.

8. Pigment mixture according to one of Claims 1 to 7, **characterised in that** the ratio of interference pigment A to interference pigment B is 99:1 to 1:99.

9. Pigment mixture according to one of Claims 1 to 8, **characterised in that** the interference pigments A and B are furthermore provided with an additional organic coating as outer layer.

10. Use of mixtures according to Claim 1 in cosmetics, surface coatings, inks, plastics, films, in security printing, in security features in documents and identity papers, for colouring seed, for colouring foods, for laser marking or in medicament coatings and for the preparation of pigment compositions and dry preparations.

11. Use according to Claim 10, **characterised in that** the mixtures according to Claim 1 are in the form of a blend with organic or inorganic dyes and/or pigments.

12. Cosmetics, surface coatings, inks, plastics, films, documents and identity papers, seed, foods or medicament coatings, pigment compositions and dry preparations comprising mixtures according to Claim 1.

## Revendications

1. Mélange de pigments d'interférence, **caractérisé en ce que** le mélange comprend au moins un pigment d'interférence A comprenant un substrat et un revêtement comprenant du fer métallique, et un pigment d'interférence B comprenant un substrat et un revêtement comprenant de l'étain métallique et de plus au moins un oxyde métallique.

2. Mélange selon la revendication 1, **caractérisé en ce que** la proportion de fer métallique dans le revêtement contenant du fer va de 10 à 100% en poids, sur la base du revêtement contenant du fer.

3. Mélange selon la revendication 1 ou 2, **caractérisé en ce que** le fer métallique est présent dans le revêtement contenant du fer en association avec FeO et/ou Fe₃O₄.

4. Mélange selon la revendication 1, **caractérisé en ce que** la proportion d'étain métallique dans le revêtement comprenant de l'étain métallique va de 0,01 to 50% en poids, sur la base du revêtement comprenant de l'étain métallique et de plus au moins un oxyde métallique.

5. Mélange selon la revendication 1 ou 4, **caractérisé en ce que** l'oxyde métallique supplémentaire est l'oxyde de titane.

6. Mélange selon l'une des revendications 1 à 5, **caractérisé en ce que** les substrats de pigments d'interférence A et B sont indépendants les uns des autres et comprennent le mica synthétique ou naturel, les phyllosilicates, le verre, SiO₂, Al₂O₃, TiO₂, le graphite et/ou BiOCl.

7. Mélange selon l'une des revendications 1 à 6, **caractérisé en ce qu'**une ou plusieurs couches comprenant des oxydes métalliques, des hydrates d'oxyde métallique, des sous-oxydes métalliques, des métaux, des fluorures métalliques, des nitrures métalliques, des oxynitrures métalliques et/ou des mélanges de ceux-ci sont en outre présents entre le revêtement comprenant du fer métallique et le substrat dans le pigment d'interférence A et/ou entre le revêtement comprenant de l'étain métallique et de plus au moins un oxyde métallique et le substrat dans le pigment d'interférence B.

8. Mélange de pigments selon l'une des revendications 1 à 7, **caractérisé en ce que** le rapport du pigment d'interférence A au pigment d'interférence B va de 99:1 à 1:99.

9. Mélange de pigments selon l'une des revendications 1 à 8, **caractérisé en ce que** les pigments d'interférence A et B possèdent en outre un revêtement organique supplémentaire comme couche externe.

10. Utilisation des mélanges selon la revendication 1 dans des produits cosmétiques, des revêtements de surface, des encres, des matières plastiques, des films, dans l'impression de sécurité, dans des dispositifs de sécurité de documents et de papiers d'identité, pour la coloration de semences, pour la coloration d'aliments, pour le marquage au laser ou dans des enrobages de médicaments et pour la préparation de compositions pigmentaires et de préparations sèches.

11. Utilisation selon la revendication 10, **caractérisée en ce que** les mélanges selon la revendication 1 sont sous forme d'un mélange avec des pigments et/ou des colorants organiques ou inorganiques.

12. Produits cosmétiques, revêtements de surface, encres, matières plastiques, films, documents et papiers d'identité, semences, aliments ou enrobages de médicaments, compositions pigmentaires et préparations sèches comprenant des mélanges selon la revendication 1.
